(19) Europäisches Patentamt
European Patent Office
Office européen des brevets

(11) Publication number : **0 064 210 B2**

# NEW EUROPEAN PATENT SPECIFICATION

(45) Date of publication of the new patent specification : 06.03.91 Bulletin 91/10

(51) Int. Cl.⁵ : **A61K 39/395, A61K 9/00**

(21) Application number : 82103266.1

(22) Date of filing : 19.04.82

(54) **Oral pharmaceutical composition containing immune globulin.**

(30) Priority : 01.05.81 US 259758

(43) Date of publication of application : 10.11.82 Bulletin 82/45

(45) Publication of the grant of the patent : 20.08.86 Bulletin 86/34

(45) Mention of the opposition decision : 06.03.91 Bulletin 91/10

(84) Designated Contracting States : CH DE FR GB IT LI

(56) References cited :
FR-A- 2 253 533
GB-A- 1 499 078
EXPERIENTIA, vol. 33, no. 1, 15th January 1977, pages 1-142; A. PELLEGRINO DE IRAL-DI: "Significance of the maillet method (Z10) for cytochemical studies of subcellular structures"

(56) References cited :
CHEMICAL ABSTRACTS, vol. 87, no. 17, 24th October 1977, page 501, no. 132159q, Columbus Ohio (USA); E.W.WILLIAMS: "Bovine IgG in the serum and organs of adult rats after oral administration"
CHEMICAL ABSTRACTS, vol. 78, no. 9, 5th March 1973, page 110, no. 53447t, Columbus Ohio (USA); A.SCOOT et al.: "Influence or orally administered porcine immunoglobulins on survival and performance of newborn colostrum-deprived pigs"
Essential Immunology I.M. Roitt (1977)
Handbook of Experimental Immunology (1979)

(73) Proprietor : MILES INC.
1127 Myrtle Street
Elkhart Indiana 46514 (US)

(72) Inventor : Hardie, Richard W.
2061 Strand Avenue
Walnut Creek California 94596 (US)

(74) Representative : Adrian, Albert, Dr. et al
c/o BAYER AG Konzernverwaltung RP
Patentabteilung
W-5090 Leverkusen 1, Bayerwerk (DE)

EP 0 064 210 B2

## Description

This invention relates to an oral pharmaceutical composition for human therapeutic use comprising human blood fractionation derived immune globulin, wherein at least 70% of the immune globulin is IgG and wherein the immune globulin is dispersed in a pharmaceutically acceptable carrier, characterized in that it contains an antidiarrheal and/or an antispasmodic.

The importance of the humoral defense system, immune globulin (IG) has long been recognized. IG preparations for therapeutic use have been available for about 30 years, however, the structure and function of IG ("gamma globulins") has only been understood in detail for a few years. Five classes of immunoglobulins are now recognized : IgA, IgG, IgM, IgE and IgD. The functions of the first four classes have been extensively researched, whereas the clinical significance of IgD is still essentially unknown. The bulk of the serum immunoglobulins (approximately 70%) are IgG, and they are the carriers of many of the body's acquired defensive functions. Like the other forms of IG, the IgG molecule consists of heavy and light polypeptide chains. Proteolytic enzymes can be used to split IgG into various fragments, called Fc, Fd and Fab fragments. For the immunoglobulin molecule to be fully functional and hence therapeutically effective, it is believed that its molecular integrity, particularly its primary or tertiary molecular structure, must be retained or, if not, its function would be impaired.

Intramuscularly injectable (IM) and intravenously injectable (IV) immune globulin preparations for parenteral administration are known. The IVIG material contains either a modified or unmodified IG molecule or both.

It has been observed that breast-fed newborn infants are better protected against gastrointestinal infection than are formula-fed infants. (Jelliffe, Amer. J. Clin. Nutr., 29,1227, 1976 ; Kleinman et al., Digestive Diseases and S.C., 24 (11) : 876 : 82, 1979 ; Beer et al., J. Invest. Dermat., 63 : 55-74,1974 ; Amman et al., Soc. for Exp. Biol. and Med., 122 : 1098-1101, 1966 ; Lourgia et al., Arch. Argent. Pediat., 72 : 109-125, 1974 ; Hilper et al., "Food and Immunology", Hambraeus L., Hanson L.A., McFarlane H. (Eds) pp. 182-196, 1977 ; Hanebery et al., Eur. J. Pediatr., 132 : 239, 1979). This can be accounted for by the presence of T and B lymphocytes, phagocytes, antibodies, complement components and other anti-bacterial substances such as lactoferrin and lysozyme. The relative importance of these elements in milk is difficult to assess although removal of cells by heating or centrifugation may lead to a significant loss in protective ability (Pitt et al., Ped. Res., 11, 906, 1977). U.S. Patent No. 4,096,244 describes a dried particulate porcine or bovine blood serum containing immunoglobulins which is acceptable to and palatable to newborn piglets when orally administered to piglets as a feed stuff component.

In U.S. Patent No. 3,975,517 a method is described wherein cows are vaccinated with a particular vaccine for coliform enteritis. Recovered milk from the so-vaccinated cows can be orally fed to newborn calves on a continuous basis.

An immune milk product containing antibodies is disclosed in U.S. Patent no. 3,911,108 and may be administered to baby pigs to protect against transmissible gastroenteritis.

Milk obtained from milk-bearing animals which have been treated with a specific mixed bacterin vaccine is described in U.S. Patent No. 3,128,230. The milk may be administered to human and lower animals for treatment of various diseases.

In U.S. Patent No. 2,607,716 there is disclosed a composition for preventing or inhibiting scours in calves, lambs, goats, pigs, rabbits, and the like. Plasma, serum, or globulin fraction of pooled blood from dairy cattle, sheep, or pigs containing immune proteins is spray-on freeze-dried and mixed with a solid Vitamin K source and partially digested milk solids. In use the mixture is mixed with water and orally administered to the animal to be treated.

DE-PS 24 09 862 describes an oral applicable immune globulin of bovin origin which cannot be regarded as the same as the human one of the present invention.

Human immune globulines were known for injectable (not oral) use (see "Rote Liste" 1976 product No. 74001-74005).

The articles in Ped. Research 14 : 591 (1980) and Ped. Research 15 : 1256-1260 are dealing with the survival of oral human immune serum globulin in low birth weight infants which are known to have enzymatic immaturity.

US-PS 4 186 192 describes a stabilized immune serum globulin and a related 1981 product information to "GAMMIMUNE®" of Cutter Biological describes its use for intravenous application.

GB-A 1 499 078 is concerned with a medicinal preparation for treating intestinal infectious diseases which contains as a major constituent IgA-containing gamma globulin, and in particular, gamma globulin derived from human serum or plasma and containing a high concentration of IgA (referred to as "IgA-rich" gamma globulin) coated with a coating ("enteric coating") capable of dissolution in the intestine to release

2

IgA

Experientia 33/1, page 138 describes the stability of orally administered bovine immuno globulines with high antibody level against pathogenic E.coli in the intestine of the infant.

It has been found that human IG may be administered orally with significant maintenance of molecular integrity. This result is surprising because degradation of the IG molecule would be anticipated to occur in the stomach by analogy with the ready degradation observed with various enzymes in vitro. As mentioned above, integrity of the IG molecule is believed to be required for therapeutic effectiveness.

The product of the present invention is an oral pharmaceutical composition for human therapeutic use comprising human blood fractionation derived immune globulin, wherein at least 70% of the immune globulin is IgG and wherein the immune globulin is dispersed in a pharmaceutically acceptable carrier, characterized in that it contains an antidiarrheal and/or an antispasmodic.

Oral administration of IG has advantages over parenteral administration. A primary advantage is the avoidance of injection, either intramuscularly or intravenously, as a means of administration and the discomforts, etc., associated therewith. An oral IG composition provides ease of administration and avoids the pain associated with parenteral administration, particularly intramuscular. Larger doses of IG may be administered orally than parenterally.

The oral pharmaceutical composition of the invention comprises orally administerable hepatitis-safe immune serum globulin in a therapeutically effective amount in a pharmaceutically acceptable carrier. The immune globulin can be prepared from human blood by fractionation in the same manner in which marterial intended for parenteral, i.e., intramuscular (IMIG) or intravenous (IVIG) use is prepared. IMIG and IVIG are well known and can be prepared by known means. For example, IMIG is commonly prepared by Cohn fractionation (Cohn et al., J. Am. Chem. Soc., 68, 459-475 [1946] ; Oncley et al., J. Am. Chem. Soc., 71, 541-550 [1949]). IVIG can be prepared by a number of methods such as ultracentrifugation (Barundern et al., Vox Sang., 7, 157-174 [1962]), pH adjustments (Koblet et al., Vox. Sang., 13, 93-102 [1967]), careful fractionation (Schneider et al., Vox Sang., 31, 141-151 [1976]), enzymatic modification (Fahey et al., J. Exper. Med., 118, 845-868 [1963] ; Kneapler et al., Vox Sang., 32, 159-164 [1977]), structural modification (Barundern et al., Monogr. Allergy, 9, 39-60 [1975]), chemical modification (Stephan, Vox Sang., 28, 422-437 [1975]) ; Masuko et al., Vox Sang., 32, 175-181 [1977]), and reduction and alkylation (Pappenhagen et al., U.S. Patent No. 3,903,262).

Other methods of fractionation to yield IG which may be used include polyelectrolyte affinity adsorption, large scale electrophoresis such as disclosed in U.S. Patent No. 4,246,085, ion exchange adsorption, and polyethylene glycol fractionation. However, any method which fractionates an immune serum globulin comprising IgG from a human source may be used in the present invention.

As the pharmaceutically acceptable carrier in accordance with the invention one may use liquid, semi-solid, e.g., pastes, or solid carriers. Particular requirements of the carrier are that it not be harmful to the recipient, that the IG be stable therein, and that the carrier not be detrimental to the IG. The IG may be combined with the carrier by solution, suspension, emulsification, admixture, encapsulation, absorption, adsorption and the like. Examples of carriers which may be used in the present invention are, by way of example and not limitation, water, fats, oils, lipids, liposomes, resins, binders and fillers, or combinations thereof. The immune globulin may, e.g. be dissolved or suspended in water, encapsulated in a liposome or adsorbed on a resin.

In some compositions a lubricant or disintegrator may be present. An important preferred characteristic of a carrier suitable in the present composition is that it protect the therapeutic effectiveness of the IG, by e.g., maintaining the integrity of the IG molecule or the activity thereof and that it facilitate delivery of active IG to the site whereat the therapeutic activity of the IG is required. By the term therapeutic effectiveness is meant that the oral composition be effective for the preventative or curative health measure for which it is orally administered similar to that for parenterally administered IG.

The oral composition of the invention may contain a stabilizing agent which protects the IG from loss of therapeutic activity by denaturation. As the stabilizing agent one may use buffers, amino acids such as glycine and lysine, carbohydrates such as dextrose, mannose, galactose, fructose, lactose, sucrose, maltose, sorbitol and mannitol, proteolytic enzyme inhibitors, or combinations thereof, in an amount sufficient to stabilize the IG in the oral composition. Also for consideration is the conjunctive use of a carrier and stabilizer to achieve the aforementioned stabilization and protective effects and controlled release preparations.

The instant product when used to treat enteric infections might also contain a bulking material such as cellulose or methylcellulose in amounts conventional in the art, e.g., about one to two grams per 100 ml.

Furthermore the present composition many contain an agent that stabilizes the immuno globulin in the digestive tract or an antidiarrheal agent such as an opium, an adsorbent powder, or an anticholinergic. Opiums useful in the invention, which are also antispasmodics (decrease time out of bowel) include codeine phosphate (dosage e.g. 15-60 mg every six hours), diphenyloxylate (e.g. 5 mg every six hours), and meperidine. An example of an adsorbent powder would be kaolin (e.g. 1-2 g every four hours).

Suitable anticholinergics are, for example, atropine (e.g.0.5-0.1 mg every 6-8 hours) and propantheline e. g. 75 mg daily in divided doses).

The product of the invention may contain an antacid, which may be administered e.g. every four to six hours, such as sodium bicarbonate (e.g. 4.4 g) magnesium oxide or hydroxide (e.g. 5.9 g), calcium carbonate (e.g. 4.5 g), magnesium trisilicate e.g. 50 g), magnesium carbonate (e.g. 63 g), and aluminum hydroxide gel (e. g. 715 ml).

The above dosages are based on current usage of the above drugs individually or in combination. The amounts of the drugs used in an oral product of the invention would be that necessary to achieve the desired results, e.g., in an antacid amount or in an anticholinergic amount. This is easily ascertained by one skilled in the art using the above guidelines. Of course, the added agent must not be detrimental to the activity of the immune globulin.

The IG should be present in the oral composition of this invention in a therapeutically effective amount. The amount of IG should, therefore, be that which will render the oral composition therapeutically active for the particular prophylactic or curative effect desired.

Preferably, the instant oral composition will contain about 1-80% IG, more preferably about 5-50%, of which not less than 70% is gamma globulin (IgG) as mentioned above. The product may contain other globulins such as IgA, IgM, IgD, and IgE. For example, Cohn Fraction II + III contains the following proportions of the above : about 8 parts IgG to 1 part each of IgA and IgM and traces of IgD and IgE.

The pH of the oral composition should be pharmaceutically acceptable and not result in destabilization of the composition. The pH of the composition should therefore be adjusted, where necessary, to within the range of about 4-8, preferably about 6-7, by addition thereto of a pharmaceutically acceptable acid or base.

It may be desirable for purposes of the invention to incorporate into the oral composition certain flavoring agents to enhance its palatability. An example of an additive for this purpose is sorbitol. However, then are of course many other flavoring agents well-known in the art which may be mixed with the oral composition in an amount sufficient to render the oral composition palatable. Generally, the flavoring agent is present in the oral composition in an amount of about 1-20% ; however, flavoring agents such as vanilla, strawberry and cherry, are usually present at a level of less than 1%.

When to be administered to adults, the material preferably is in the form of a tablet.

Generally immune globulins for intramuscular administration contain a mercury compound. The orally administerable material according to the invention should be free of any mercury compound.

The orally administerable immune globulin compositions according to the invention may be prepared by a method which comprises :

(a) mixing an orally-administerable immune globulin with a pharmaceutically acceptable carrier and, optionally a flavoring agent and/or other additives,

(b) adjusting the pH of the mixture to about 4-8 and

(c) rendering the mixture in a form suitable for oral administration.

A typical formulation of an oral-pharmaceutical composition in accordance with the invention is, by way of example and not limitation, an aqueous solution having the following composition :

| | |
|---|---|
| IG concentration | 5 - 20 g/100 ml. |
| pH | 6 - 7 |
| Sterility, pyro- genicity, safety | pharmaceutically acceptable |
| Purity of IG | NLT* 90% Gamma Globulin |
| Carbohydrate such dextrose, maltose, sorbitol | 1 - 20 g/100 ml. |

* NLT = Not less than

The starting material for the above formulation is the globulin fraction isolated from human blood plasma by Cohn fractionation (Cohn et al., ibid.), which is known to be hepatitis-safe. (Cohn Fraction II + III paste, the hepatitis safety of which is not known, may be rendered so by methods known in the art such as by heat pasteurization in the presence of a stabilizer.) Dry Immune Globulin (human) or Cohn Fraction II paste

4

is suspended in a carbohydrate solution such that its protein concentration is 5-20%. The temperature of the suspension is maintained at not more than 5°C. The solution pH value is adjusted to about 6-7 by addition of a pharmaceutically acceptable acid or base, and the solution is clarified. The solution is sterile filtered and filled into appropriate bottles. Each bottle contains 1.0 gram of protein and 0.5 gram of carbohydrate. The final container bottles are plug frozen, lyophilized, and stored at 2° to 8°C.

## Oral Administration of IG

Patients and protocol – Seven thriving formula-fed 4-13 week old immature infants (birth weight .86-1.46 kg. ; weight at study 1.36-1.7 kg.) were selected from a transitional nursery population. None received breast milk in the two weeks prior to this study. A modified immune globulin (MIG) prepared according to U.S. Patent Nos. 3,903,262 and 4,186,192 in doses of from 1-8 ml/kg/day in divided doses was administered orally in formula feedings for 5 consecutive days to six infants. One infant served as control. All stools were collected and saved for each 24 hour period beginning with the day before MIG feedings began and continuing for 2 days after they ended. The samples were then frozen at – 20°C for later determinations of immunoglobulin content and opsonic activity.

Coproantibodies – The frozen stool samples were quantitatively removed from the diapers, lyophylized, ground into powder and weighed. Ten ml. of phosphate buffered saline (pH 7.2) was added for each gram of dried stool, mixed for 30 minutes at room temperature and then spun at $20,000 \times g$ at 4°C for 30 minutes. The supernatant was removed, sterile filtered, and stored at –70°C until needed. Quantitative immunoglobulin G, A and M levels on these samples were performed by radial immunodiffusion (Meloy Laboratories, Springifeld, VA and Kallestad Laboratories, Chaska, Minn.). Immunoelectrophoresis was performed according to the method of Scheidegger, Int. Arch. Allergy, 7, 103 (1955) with MIG or stool extract in the wells. After 2 hours in the chamber, antisera to IgG, IgA, and IgM were added to the troughs.

Opsonic activity as measured by neutrophil chemiluminescence – The MIG employed contained high antibody titer to Group B streptococcus and this organism was chosen as the target for opsonization. Type III Group B streptococcus (SS-893, supplied by the Communicable Disease Center, Atlanta, Georgia) was prepared and standardized according to the method of Hemming et al., J. Clin. Invest., 58, 1379 (1976).

The Group B streptococci were opsonized by mixing 0.4 ml with 0.1 ml of stool extract and rotating for 40 minutes at 37°C. The organisms were then washed twice in phosphate buffered saline, centrifuged and diluted to original volume before being used immediately in the chemiluminescence assay.

Chemiluminescence (CL) was performed in a liquid scintillation counter (Beckman LS 8000) in the single photon count mode with the reaction mixtures containing 0.7 ml of PMN ($2.5 \times 10^6$/ml) and 0.3 cc. of opsonized Group B streptococci ($5 \times 10^8$ to $1 \times 10^9$ colony forming units/ml.). All reaction vials were kept at 37°C in a Dubnoff shaking water bath and removed only for the brief time required for counting.

Clinical observations – No adverse effects of the oral MIG feedings were noted during the course of the study. There was no increased regurgitation of feedings, diarrhea, or other alteration in stool pattern. All infants remained clinically well and continued to gain weight.

Stool Immunoglobulins – The MIG employed contained 14 mg/dl. of IgG. Trace quantities of both IgM and IgA immunoglobulins were found in all stool samples, including the control samples collected on each infant before and after the MIG feedings and in the control infant not given MIG. Levels of IgM and IgA did not rise during the MIG feedings.

No infant had measurable IgG in 24 hours stool samples collected before initiation of immunoglobulin feedings, further, in each case, the stool IgG levels declined to negligible amounts within 48 hours after the last feeding. IgG was found in the stools of all six infants fed MIG. This IgG ranged from 3 to 72 mg/24 hours and represents 4-12% of the oral dose. Increasing doses of oral MIG were associated with higher amounts of IgG excreted per day, suggesting a linear relationship between the amount ingested and the amount recovered.

Opsonic studies – Normal granulocytes ($1.75 \times 10^6$) were mixed with Group B streptococcus exposed to saline, MIG or stool supernatants from an MIG-fed infant and the chemiluminescence produced is measured over time. The peak response in cpm per $2 \times 10^6$ cells is used as an index of opsonic activity. This is generally reached at seven minutes after addition of PMNs to the reaction vial. There was then a rapid falloff in counts over the subsequent 21 minutes., The curves for MIG, stools, and saline are similar in configuration, except for the peak CL achieved.

Stool samples with IgG levels greater than 100 mg/dl. uniformly supported chemiluminescence (CL) and were effective opsonins for Group B streptococcus at titers ranging up to 1/8 or 1/16. Stool samples with IgG levels less than 100 mg/dl. did not opsonize Type III Group B streptococcus and therefore were poorly supportive of CL. This was also true for stools from the control infant as well as for all stool samples collected prior to MIG feedings and 48 hours after MIG feedings.

With respect to CL counts and their relationship to stool IgG levels, intensity of CL response did not relate to the IgG level in a linear fashion. With respect to chemiluminescence response on stool samples with antibody levels both above and below 100 mg/dl., on undiluted MIG and on saline controls, the average CL counts of those samples with coproantibody levels in excess of 100 mg/dl. was $69 \times 10^3$ compared with $69 \times 10^3$ for samples with levels less than 100 ml/dl. The difference was highly significant ($p < .001$).

The difference between the high IgG samples and the saline controls ($40 \times 10^3$) was also highly significant ($p < .001$) as was that between MIG and saline controls ($p < .001$). No statistical difference in CL was found between saline controls and samples with less than 100 mg/dl. of IgG or between samples with greater than 100 mg/dl. of IgG and MIG.

In studies employing a rabbit ileal loop model, Zinkernagel et al., in Med. Microbiol. Immunol., 162, 1 (1975) showed that passive immunization with, i,e., injection into a loop of rabbit intestine of bovine immunoglobulin (50 mg. of IgG per loop) was effective in decreasing viability of several strains of human enterophathogenic E. coli.

The above infant study demonstrates that the infants stool contained significant quantities of undigested and intact IgG and that this coproantibody retained significant opsonic activity for Type III Group B streptococci. Oral IG, therefore, may be used in prevention or treatment of enteric infections, e.g., E. coli, V. cholera, S. typhosa or intoxications, e.g. infantile botulism, since intact IgG with opsonic activity persisted in the gastro-intestinal tract and thus is available to function in such prevention or treatment.

## Claims

1. An oral pharmaceutical composition for human therapeutic use comprising human blood fractionation derived immune globulin, wherein at least 70% of the immune globulin is IgG and wherein the immune globulin is dispersed in a pharmaceutically acceptable carrier, characterized in that it contains an antidiarrheal and/or an antispasmodic.

2. A composition according to claim 1, characterized in that the carrier is a liquid and the composition has a pH of 4-8.

3. A composition according to claim 1, characterized in that the carrier is a solid.

4. A composition according to claim 3, characterized in that it is in the form of a tablet.

5. A composition according to claims 1 to 4, characterized in that it further contains a stabilizing agent selected from the group consiting of buffers, amino acids, carbohydrates, anticholinergics, antacids and proteolytic enzyme inhibitors.

6. A composition according to claims 1 to 5, characterized in that the amount of orally administerable immune globulin in the composition is 1-80% by weight, preferably 5-50% by weight.

7. A composition according to claims 1 to 6, characterized in that it further contains a flavoring agent.

## Ansprüche

1. Eine orale pharmazeutische Zusammensetzung für die Verwendung in der Humantherapie, umfassend von einer Humanblutfraktionierung abgeleitetes Immunglobulin, in welcher wenigstens 70% des Immunglobulins IgG ist und in welcher das Immunglobulin in einem pharmazeutisch annehmbaren Träger dispergiert ist, dadurch gekennzeichnet, daß es ein Antidiarrhoikum und/oder ein Antispasmatikum enthält.

2. Zusammensetzung gemäß Anspruch 1, dadurch gekennzeichnet, daß der Träger flüssig ist und die Zusammensetzung einen pH-Wert von 4 bis 8 hat.

3. Zusammensetzung gemäß Anspruch 1, dadurch gekennzeichnet, daß der Träger fest ist.

4. Zusammensetzung gemäß Anspruch 3, dadurch gekennzeichnet, daß er in Form einer Tablette vorliegt.

5. Zusammensetzung gemäß Ansprüchen 1 bis 4, dadurch gekennzeichnet, daß sie weiterhin ein Stabilisierungsmittel enthält, welches ausgewählt ist aus der Gruppe bestehend aus Puffern, Aminosäuren, Kohlenhydraten, Anticholinergika, Antacida und proteolytischen Enzyminhibitoren.

6. Zusammensetzung gemäß Ansprüchen 1 bis 5, dadurch gekennzeichnet, daß die Menge des oral verabreichbaren Immunglobulins in der Zusammensetzung 1 bis 80 Gew.-%, vorzugsweise 5 bis 50 Gew.-% beträgt.

7. Zusammensetzung gemäß Ansprüchen 1 bis 6, dadurch gekennzeichnet, daß es weiterhin einen Geschmacksstoff enthält.

**Revendications**

1. Composition pharmaceutique orale à usage thérapeutique humain, comprenant une immunoglobuline obtenue par fractionnement de sang humain, dans laquelle au moins 70% de l'immunoglobuline consistent en IgG et dans laquelle l'immunoglobuline est dispersée dans un support acceptable du point de vue pharmaceutique, caractérisée en ce qu'elle contient une substance anti-diarrhéique et/ou un agent antispasmodique.

2. Composition suivant la revendication 1, caractérisée en ce que le support est un liquide et la composition a un pH de 4-8.

3. Composition suivant la revendication 1, caractérisée en ce que le support est une matière solide.

4. Composition suivant la revendication 3, caractérisée en ce qu'elle est sous la forme d'un comprimé.

5. Composition suivant les revendications 1 à 4, caractérisée en ce qu'elle contient en outre un agent stabilisant choisi entre des tampons, des amino-acides, des glucides, des anticholinergiques, des antacides et des inhibiteurs d'enzymes protéolytiques.,

6. Composition suivant les revendications 1 à 5, caractérisée en ce que la quantité d'immunoglobuline administrable par voie orale dans la composition va de 1 à 80% en poids, de préférence de 5 à 50% en poids.

7. Composition suivant les revendications 2 à 5, caractérisée en ce qu'elle contient en outre une substance aromatique.